Europäisches Patentamt

European Patent Office

Office européen des brevets

(11) Publication number: **0 485 347 B1**

## EUROPEAN PATENT SPECIFICATION

(45) Date of publication of patent specification: **14.09.94**

(51) Int. Cl.⁵: **C07K  15/00**, C12N 15/62, C12N 15/40, G01N 33/576

(21) Application number: **91830479.1**

(22) Date of filing: **04.11.91**

(54) Recombinant hepatitis delta antigen, process for the purification and use thereof.

(30) Priority: **05.11.90 IT 6786590**

(43) Date of publication of application:
**13.05.92 Bulletin  92/20**

(45) Publication of the grant of the patent:
**14.09.94 Bulletin  94/37**

(84) Designated Contracting States:
**AT BE CH DE DK ES FR GB GR IT LI LU NL SE**

(56) References cited:
**EP-A- 0 175 261**
**EP-A- 0 251 575**

**J. GEN. VIROL. vol. 71, 1990, pages 1339 - 1345; T. B. MACNAUGHTON: 'Stable expression of hepatitis delta virus'**

**J. CLIN. MICRO. vol. 27, 1989, pages 2222 - 2225; J. PUIG: 'Development of an enzyme immunoassay'**

**J. VIROL. vol. 62, no. 6, 1988, pages 1855 - 1861; M. KUO: 'Molecular cloning of hepatitis delta virus RNA'**

**J. VIROL. vol. 64, no. 9, 1990, pages 4053 -**

**4058; J. LIN: 'Characterisation of hepatitis delta antigen'**

(73) Proprietor: **SORIN BIOMEDICA S.p.A.**
**Via Crescentino**
**I-13040 Saluggia (Vercelli) (IT)**

(72) Inventor: **Bonelli, Fabrizio**
**c/o Sorin Biomedica S.p.A.**
**I-13040 Saluggia (VC) (IT)**
Inventor: **Calogero, Raffaele**
**c/o Sorin Biomedica S.p.A.**
**I-13040 Saluggia (VC) (IT)**
Inventor: **Boniolo, Antonio**
**c/o Sorin Biomedica S.p.A.**
**I-13040 Saluggia (VC) (IT)**

(74) Representative: **de Simone, Domenico et al**
**Ing. Barzanò & Zanardo Roma S.p.A.**
**Via Piemonte 26**
**I-00187 Roma (IT)**

Rank Xerox (UK) Business Services
(3. 10/3.0 9/3.3.3)

## Description

Technical field of the invention

This invention relates to Hepatitis Delta Viral antigen (HDAg) expressed from host cells transformed with vectors comprising HDAg coding sequences, to a process for the purification thereof in an antigenic form and to its use in the diagnostic field. More specifically, the invention relates to HDAg-like polypeptides expressed from recombinant vectors wherein the expressed polypeptide functions in diagnostic and therapeutic applications in a manner characteristic of the HDAg polypeptide found within liver cells infected with the Hepatitis Delta Virus (HDV).

Background art

HDV is a major human pathogen that was discovered by Rizzetto and coworkers in Hepatitis B Virus (HBV)-infected patients experiencing severe liver disease (Rizzetto et al., Gut, 18:997-1003, 1977). HDV depends on HBV for the provision of a coat and accordingly has only been found in patients also infected with HBV. The HDV genome consists of a circular, single stranded RNA approximately 1.7 kb in length with the ability to fold on itself by intramolecular base pairing to form a double-stranded rod-like structure (Kos et al., Nature 323:558, 1986). Recent studies have demonstrated that HDV replication is independent of the presence of HBV (Taylor et al., J. Virol. 61:2891, 1987).

It has been shown that an antigenic protein, denominated HDAg is encoded by one of the HDV open reading frames (ORF5) and results to be indispensable for replication of the HDV genome. The identification and partial characterization of ORF5 is described in the EP Applications No. 0234050 and No. 0251575. The activity of ORF5 can be supplied in trans, by the diffusible HDAg and does not necessarily require the physical proximity of ORF5 to the replication control regions of the virus. The role of HDAg has been studied extensively with respect to its role in viral replication (Luo et al., J. Virol. 64:1021, 1990; Hsieh et al., J. Virol. 64:3192, 1990; Chao et al., J. Virol. 64:5066, 1990). HDAg has been found to exist in a 24 kd form and a 27 kd form (Bergmann et al., J. Infect. Dis. 154:702, 1986), with the larger form (214 amino acids) representing a 19 amino acid carboxyl-terminal extension of the smaller (195 amino acids) form (Taylor, Cell 61:371, 1990). The larger form which may actually inhibit viral replication (Taylor, supra) may arise from conversion of a UAG termination codon for the smaller HDAg to UGG, permitting synthesis of the larger variant polypeptide (Luo et al., supra; Taylor, supra).

The diagnosis of HDV infection is usually established by detection of corresponding total and/or M (IgM) immunoglobulins directed against HDAg. To detect anti-HDAg immunoglobulin activity various blocking and competitive inhibition enzyme immunoassays have been developed (Crivelli et al., J. Clin. Microbiol. 14:173, 1981; Purcell and Gerin, Virology, 2nd Ed., Fields et al. Eds., 2275-2283, 1990; Farci et al., J. Am.Med.Assoc. 255:1443, 1986). All of these immunoassays make use of the natural antigen that can be extracted from human- or chimpanzee-infected liver.

Unfortunately, the natural antigen is present in the hepatocytes at very low concentration and is extremely unstable under the relatively harsh conditions that may accompany isolation and purification of the antigen (e.g. , 6M guanidinium HCl, 8M urea). Such instability may lead to formation of relatively low molecular weight peptides with reduced immunodiagnostic or immunotherapeutic utility compared to a full-length or nearly full-length HDAg.

Kuo M.Y.P. et al. J. Virol. 1988, p. 1855-1861 discloses a protein coded by a β-galactosidase-HDAg gene which when expressed in E. Coli, may be recovered from cells in a single step by immunoaffinity chromatography. As it will appear from the following description the present invention refers to a fused gene with a β-gal portion much shorter, a HDAg sequence longer and a tet sequence at the C-terminal.

A high level of expression of recombinant polypeptides In E.coli frequently results in formation of cytoplasmic granules observable with a phase-contrast microscope. Such insoluble granules have been termed "inclusion bodies", and are thought to represent macromolecular complexes of the heterologous polypeptide and the bacterial outer membrane (Frankel et al., Proc. Natl. Acad. Sci USA 88:1192, 1991). The inclusion bodies frequently are used as a source of expressed polypeptide, since the inclusion bodies are readily pelleted by centrifugation and provide a convenient source of polypeptide. However, to obtain soluble protein, the inclusion bodies must be solubilized under relatively harsh conditions such as 5-7 M guanidine HCl, 6-8 M urea, sodium dodecyl sulfate (SDS), alkaline pH, and/or acetonitrile/propanol Thus, isolation of recombinant HDAg or HDAg-like polypeptides from inclusion bodies yields peptides having reduced immunodiagnostic and immunotherapeutic utility in comparison to native HDAg or to expressed HDAg.

2

Disclosure of the invention

Figure 1      shows a restriction map of pSORF5.

Figure 2      shows a restriction map of pHBc27.

Figure 3      shows a restriction map of pSORINδ.

Vector construction generally employs techniques now well understood by those skilled in the art. Site-specific DNA cleavage is performed by means of suitable restriction enzymes under conditions generally specified by the manufacturer of commercially available restriction enzymes (see for example the New England Biolabs Product Catalogue). Sticky end cleavage fragments may be blunt ended using E.coli DNA polymerase 1 (Klenow fragment) in the presence of appropriate deoxynucleotide triphosphates (dTNPs) using incubation conditions appropriate to the polymerase. The polymerase fills in 5' protruding ends. Alternatively, treatment with S1 nuclease may be used, with resultant hydrolysis of any single stranded DNA portion. Ligations, whether with sticky ends or blunt ends, are carried out using standard buffer and temperature conditions with T4 DNA ligase. Vector fragments are often treated with bacterial alkaline phosphatase in order to remove the 5' phosphate and thus prevent religation of the vector. Ligation mixtures may be transfected into suitable hosts, such as E.coli, and successful transformants selected by antibiotic resistance or other appropriate means, then screened for the correct orientation of insert.

The term transformation includes, without limitation, known methods for transformation of host cells by nucleic acid uptake, microinjection, electrotransformation, electroporation, pellet bombardment or any other suitable method for transfer of exogeneous nucleic acid into host cells (see, e.g. Sambrook et al., Molecular Cloning A. Laboratory Manual, 2nd Ed., Cold Spring Harbor Laboratory Press, 1989).

In contrast to isolation of expressed polypeptides from the commonly used inclusion bodies, applicants have discovered in particular that undenatured HDAg-like polypeptide may be isolated in multimeric form from the cytosolic fraction of bacterial cells using a simple one-step ultracentrifugation procedure. This particular discovery may be generalized as follows. As used herein, the terms "cytosol" or "cytosolic fraction" refer to prokaryotic or eukaryotic cell cytoplasm exclusive of membranes and membrane-bound organelles. That is, the cytosol or cytosolic fractions is defined by the intracellular regions between membrane-bounded organelles. Polypeptides in the cytosol generally constitute "soluble" polypeptides, though such polypeptides may exist in monomeric or multimeric forms and may be capable of organizing as aggregates or particles when in the multimeric form. Such cytosolic aggregates, however, require relatively high-speed centrifugation for purification and generally do not co-purify with membrane-bound organelles or other cellular structures, such as inclusion bodies, which are readily pelleted by low speed centrifugation.

The invention involves the finding that multimeric aggregates of HDAg recombinant polypeptide have an antigenicity substantially comparable to the antigenicity of HDAg antigen of HDV-infected human liver cells. The aggregate is recovered from host cells which have been transformed with a recombinant vector able to express HDAg polypeptide. The aggregate may further contain HDV RNA bound to the polypeptide. In the case where the host cells are bacterial cells, the aggregates may be recovered by separating such aggregates from cell membrane material and cell inclusion bodies resulting, from sonication or any other method of disrupting the bacteria. This separation of aggregate from cell membrane material and cell inclusion bodies may be accomplished by centrifugation. Subsequent to the separation by centrifugation, the aggregates may also be separated substantially from cytosolic material by ultracentrifugation such as sucrose gradient fractionation.

The present invention solves the problems referred to by providing recombinant DNA molecules able to express after transformation in a suitable host recombinant HDAg with an antigenicity substantially comparable to the antigenicity of HDAg of HDV from infected human liver cells,namely being able to raise antibody against HDAg. The present invention provides also a process for the purification of the HDAg with an antigenicity substantially comparable to the antigenicity of HDAg of HDV front infected human liver cells and its use in the diagnostic field.

In a preferred embodiment, the host cells may be transformed with a recombinant expression vector carrying the coding information for HDAg-like recombinant polypeptides, containing a first region substantially homologous to substantially intact HDV antigen and an amino terminal second region contiguous to the first region and a carboxyl terminal third region contiguous to the first region, wherein the first region is HDAg antigenic and the second region and third region are each substantially heterologous to HDV antigen. The second region and third region may each be coded by nucleic acid sequences substantially heterologous to related HDV RNA. Where the host cells are bacterial cells, preferably E.coli, the aggregates may be recovered by separating the aggregates from cell membrane material and cell inclusion bodies and subsequently separating the aggregates from the cytosol of disrupted host cells by either ultracentrifugation on sucrose gradient or sequential immunoaffinity chromatography specific to the second region and third region. Preferably said second region comprises a region from the E. coli beta-galattosidase coding region and said third region comprises a coding region from pBR322 plasmid. According to a preferred embodiment, the HDAg-like recombinant polypeptide is, or is substantially homologous to, B-HDAg-T, whose aminoacid sequence is shown in Table 1.

Table 1 Nucleic acid and amino acid sequences of

B-HDAg-T (-): beta-galactosidase; (_): linker; (>):

HDAg; (*): pBR322 tet gene

```
           10        20        30        40        50        60
            |         |         |         |         |         |
ATGACCATGATTACGGATTCACTGGAATTGATCCCCATGAGCCGGTCCGAGTCGAGGAAG

   M   T   M   I   T   D   S   L   E   L   I   P   M   S   R   S   E   S   R   K
   --------------------------         _____  . .   >>>>>>>>>>>>>>>>>>>>>>>


           70        80        90       100       110       120
            |         |         |         |         |         |
AACCGCGGAGGGAGAGAAGAGATCCTCGAGCAGTGGGTGGCCGGAAGAAAGAAGTTAGAG

   N   R   G   G   R   E   E   I   L   E   Q   W   V   A   G   R   K   K   L   E
   >>>>>>>>>>>>>>>>>>>>>>>>>>>>>>>>>>>>>>>>>>>>>>>>>>>>>>>>>>>>>>>>
```

4

```
            130         140         150         160         170         180
             |           |           |           |           |           |
GAACTCGAGAGAGACCTCCGGAAGACAAAGAAGAAACTCAAAAAGATTGAGGACGAAAAT

    E   L   E   R   D   L   R   K   T   K   K   K   L   K   K   I   E   D   E   N
>>>>>>>>>>>>>>>>>>>>>>>>>>>>>>>>>>>>>>>>>>>>>>>>>>>>>>>>>>>>>>>>>>>>>>>>>>>>>

            190         200         210         220         230         240
             |           |           |           |           |           |
CCCTGGCTGGGGAACATCAAAGGAATTCTCGGAAAGAAGGATAAGGATGGAGAGGGGGCT

    P   W   L   G   N   I   K   G   I   L   G   K   K   D   K   D   G   E   G   A
>>>>>>>>>>>>>>>>>>>>>>>>>>>>>>>>>>>>>>>>>>>>>>>>>>>>>>>>>>>>>>>>>>>>>>>>>>>>>>>

            250         260         270         280         290         300
             |           |           |           |           |           |
CCCCCGGCGAAGAGGGCCCGAACGGACCAGATGGAGGTAGACTCCGGACCTAGGAAGAGG

    P   P   A   K   R   A   R   T   D   Q   M   E   V   D   S   G   P   R   K   R
>>>>>>>>>>>>>>>>>>>>>>>>>>>>>>>>>>>>>>>>>>>>>>>>>>>>>>>>>>>>>>>>>>>>>>>>>>>>>>>

            310         320         330         340         350         360
             |           |           |           |           |           |
CCTCTCAGGGGAGGATTCACCGACAAGGAGAGGCAGGATCACCGACGAAGGAAGGCCCTC

    P   L   R   G   G   F   T   D   K   E   R   Q   D   H   R   R   R   K   A   L
>>>>>>>>>>>>>>>>>>>>>>>>>>>>>>>>>>>>>>>>>>>>>>>>>>>>>>>>>>>>>>>>>>>>>>>>>>>>>>>

            370         380         390         400         410         420
             |           |           |           |           |           |
GAGAACAAGAAGAAGCAGCTATCGGCGGGAGGCAAGAACCTCAGCAAGGAGGAAGAAGAG

    E   N   K   K   K   Q   L   S   A   G   G   K   N   L   S   K   E   E   E   E
>>>>>>>>>>>>>>>>>>>>>>>>>>>>>>>>>>>>>>>>>>>>>>>>>>>>>>>>>>>>>>>>>>>>>>>>>>>>>>>

            430         440         450         460         470         480
             |           |           |           |           |           |
GAACTCAGGAGGTTGACCGAGGAAGACGAGAGAAGGGAAAGAAGAGTAGCCGGCCCGCCG

    E   L   R   R   L   T   E   E   D   E   R   R   E   R   R   V   A   G   P   P
>>>>>>>>>>>>>>>>>>>>>>>>>>>>>>>>>>>>>>>>>>>>>>>>>>>>>>>>>>>>>>>>>>>>>>>>>>>>>>>

            490         500         510         520         530         540
             |           |           |           |           |           |
.GTTGGGGGTGTGATCCCCCTCGAAGGTGGATCGAGGGGAGCGCCCGGGGGCGGCTTCGTC

    V   G   G   V   I   P   L   E   G   G   S   R   G   A   P   G   G   G   F   V
>>>>>>>>>>>>>>>>>>>>>>>>>>>>>>>>>>>>>>>>>>>>>>>>>>>>>>>>>>>>>>>>>>>>>>>>>>>>>>>
```

5

```
        550       560       570       580       590       600
         |         |         |         |         |         |
   CCCAGTCTGCAGGGAGTCCCGGAGTCCCCCTTCTCTCGGACCGGGGAGGGGCTGGACATC

     P   S   L   Q   G   V   P   E   S   P   F   S   R   T   G   E   G   L   D   I
     >>>>>>>>>>>>>>>>>>>>>>>>>>>>>>>>>>>>>>>>>>>>>>>>>>>>>>>>>>>>>>>

        610       620       630       640       650       660
         |         |         |         |         |         |
   AGGGGAAACCGGGGATTTCCATGGGATATACTCTTCCCAGCCGATCCGCCCTTTTCTCCC

     R   G   N   R   G   F   P   W   D   I   L   F   P   A   D   P   P   F   S   P
     >>>>>>>>>>>>>>>>>>>>>>>>>>>>>>>>>>>>>>>>>>>>>>>>>>>>>>>>>>>>>>>

        670       680       690       700       710       720
         |         |         |         |         |         |
   CAGAGGGGTCCTCTACGCCGGAGCCATCGTGGCCGGCATCACCGGCGCCACAGGTGCGGT

     Q   R   G   P   L   R   R   S   H   R   G   R   H   H   R   R   H   R   C   G
     >>  *********************************************************

        730       740       750       760       770       780
         |         |         |         |         |         |
   TGCTGGCGCCTATATCGCCGACATCACCGATGCGGAAGATCGGGCTCGCCACTTCGGGCT

     C   W   R   L   Y   R   R   H   H   R   C   G   R   S   G   S   P   L   R   A
     ***********************************************************

        790       800       810       820       830       840
         |         |         |         |         |         |
   CATGAGCGCTTGTTTCGGCGTGGGTATGGTGGCAGGCCCCGTGGCCGGGGGACTGTTGGG

     H   E   R   L   F   R   R   G   Y   G   G   R   P   R   G   R   G   T   V   G
     ***********************************************************

        850       860       870       880       890       900
         |         |         |         |         |         |
   CGCCATCTCCTTGCATGCACCATTCCTTGCGGCGGCGGTGCTCAACGGCCTCAACCTACT

     R   H   L   L   A   C   T   I   P   C   G   G   G   A   Q   R   P   Q   P   T
     ***********************************************************

        910       920       930
         |         |         |
   ACTGGGCTGCTTCTTAATGCAGGAGTCGCATAA

     T   G   L   L   L   N   A   G   V   A   -
     *********************************
```

According to the present invention a method is furnished to allow expression of a nearly full-length HDAg and recovery of the expressed polypeptide under conditions substantially preserving the conformational epitopes of the antigen, preferably without use of denaturing agents, said HDAg having an antigenicity comparable to the intact natural antigen present in infected human or chimpanzee liver cells in the sense that the recombinant HDAg-like polypeptide is cross-reactive with and has a similar affinity index as the naturally derived HDAg. Thus, the recombinant HDAg-like polypeptide of the present invention is particularly useful in immunoassay of the HDAg in body tissue and fluids or antibodies in serum specific to such HDAg. The recombinant HDAg-like polypeptide is useful as a standard for such assays as well as

6

other reagents as more fully discussed below.

In a preferred embodiment of the invention the HDAg-like polypeptide is encoded by an expression vector containing appropriate expression and other control elements as well as sequence encoding all but the carboxyl-terminal six amino acids of the large form of HDAg. In a specific embodiment of the invention the said expression vector is the plasmid pSORINδ, deposited at DSM N. 6774.

According to this embodiment of the invention, E.coli cells expressing HDAg-like polypeptide from pSORINδ are centrifuged to form a cell paste, resuspended in lysis buffer, and sonicated under conditions generally disrupting the outer bacterial membrane while generally leaving the inclusion bodies intact. Any other known method for disruption of cells while leaving intact the inclusion bodies may be employed without departing from the scope of the present invention. Following cellular disruption, the cellular membranes, inclusion bodies and other cellular debris were pelleted by low speed centrifugation (e.g. 10.000 rpm in a JA17 Beckman rotor for 30 min. at 4.C). The supernatant containing the cytosolic fraction with soluble polypeptides is applied to the top of a 10-50% sucrose gradient and subjected to a 16-hour ultracentrifugation run. Thereafter, the expressed HDAg-like polypeptide is isolated from the bottom of the gradient in the form of multimeric aggregates. While the HDAg-like polypeptide isolated from sucrose gradients is eminently suited to immunodiagnostic and other applications, it is to be understood that other centrifugation media known to those skilled in the art, such as caesium sulfate and caesium chloride, may be employed for ultracentrifugation of HDAg-like polypeptide multimers.

As an alternative to isolation of HDAg-like polypeptide by ultracentrifugation, the HDAg-like polypeptide may be purified by immunoaffinity chromatografy. The applicants have developed a double column procedure that exploits polypeptide sequence, heterologous to ORF5, that is contiguous with the ORF5 amino acid sequence at the amino terminal and carboxyl terminal portions of the expressed polypeptide. That, is, a monoclonal antibody (MAB-B) was raised to the non-ORF5 amino terminal portion of the expressed polypeptide. In like manner, a monoclonal antibody (MAB-T) was raised to the non-ORF5 carboxyl terminal portion of the expressed polypeptide. MAB-B was used on a first immunoaffinity column, and MAB-T was used on a second immunoaffinity column.

The same cellular lysate material as was applied to the top of the sucrose gradients was applied to the first immunoaffinity column. The MAB-B-bound material was eluted and applied to the second column. Only expressed polypeptide having both amino-terminal and carboxyl-terminal portions recognized by MAB-B and MAB-T, respectively, will be retained by the second column. Such polypeptide material will include the entire ORF5 encoded by pSORINδ, since the ORF5 amino acid sequence is located between the heterologous amino acid sequences recognized by MAB-B and MAB-T. Following elution from the second column, the expressed polypeptide may be employed in a variety of immunodiagnostic and immunotherapeutic applications.

The purified expressed HDAg-like polypeptide is useful in a variety of immunoassay formats well known in the art. The invention includes use of the purified multimeric aggregates as described above for detection of analyte antibodies or analyte antigens in patient samples. As an illustration, analyte antibodies may be detected such as total immunoglobulin (Ig) or as immunoglobulin M (IgM).

As an example of immunoassay for anti-HDAg Ig, microtiter plates may be coated with MAB-T using standard procedures. The expressed polypeptide then may be added to the plates under conditions promoting binding of the polypeptide to MAB-T. Having attached the expressed HDAg-like polypeptide to the solid phase, patient serum and enzyme-labelled anti-HDAg polyclonal antibody may be added to the plates in a competitive immunoassay format well known to those skilled in the art. The amount of enzyme-labelled anti-HDAg polyclonal antibody bound to the plate is a measure of the amount of anti-HDAg Ig in the serum sample. In an alternative embodiment, biotinilated HDAg may be bound to the solid phase via streptavadin.

As an illustration of immunoassay for anti-HDAg IgM, microtiter plates may be coated with anti-human IgM monoclonal antibody according to standard methods. Patient serum is then added to the plate to sequester patient IgM to the solid phase. These solid phase patient IgM antibodies are then reacted simultaneously or sequentially with HDAg-like polypeptide and with enzyme-labelled anti-HDAg polyclonal antibody. The amount of enzyme label that becomes bound to the solid phase is a measure of the amount of anti-HDAg IgM in the patient sample. In an alternative embodiment, enzyme-labelled MAB-T may be used in place of the enzyme-labelled anti-HDAg polyclonal antibody.

Detection of analyte HDAg in a patient sample may be accomplished with a microtiter plate or other solid phase having attached an anti-HDAg antibody. Patient sample is added to the plate for reaction. The solid phase bound HDAg from the patient sample is then reacted with a labelled anti-HDAg antibody.

Detection of analyte HDAg in a patient sample may also be accomplished in any of the competitive binding immunoassay formats known to those skilled in the art, utilizing the purified HDAg polypeptide as described above. In such formats, HDV antigen present in the patient sample competes with the expressed HDAg-like polypeptide for binding to an anti-HDAg antibody.

The following examples are intended to illustrate but not to limit the invention.

EXAMPLE 1 Construction of Plasmid pSORF5

Total RNA from a biopsy of HDV-infected human liver was extracted as described (Chirgwin, Biochemistry 18:5294, 1979). A 20 ug sample of this RNA was analyzed in Northern blot format (Thomas, Proc. Natl. Acad. Sci. 77:5201, 1980), by hybridization to the oligonucleotide probe M316 (3' CGA ACG GAC CAG ATG GAG GTA GAC TCC GGA CCT AGG AAG AG 3') (data not shown). 18 ug of total RNA were used as template for first strand cDNA synthesis as described (Sambrook et al., supra), using as primer the oligonucleotide M314 (5' ATG AGC CGG TCC GAG TCG AGG AAG 3'). The cDNA reaction mixture was used in PCR amplification experiments (Kawasaki, In: Innis et al., eds., PCR Protocols; A Guide to Methods and Applications, Academic Press, pp. 21-27), using as primers the oligonucleotides M314 and M315 (5' TCA CTG GGG TCG ACA ACT CTG GGG AGA 3'). The PCR amplification was performed with a protocol of 35 cycles, each cycle consisting of one min. at 95C, one min. at 50C, and three min. at 65C. The amplification reaction produced two major bands: one of 300 bp and the other of 640 bp (data not shown). Southern blot analysis revealed that only the 640 bp band hybridized with oligonucleotide M316 (data not shown).

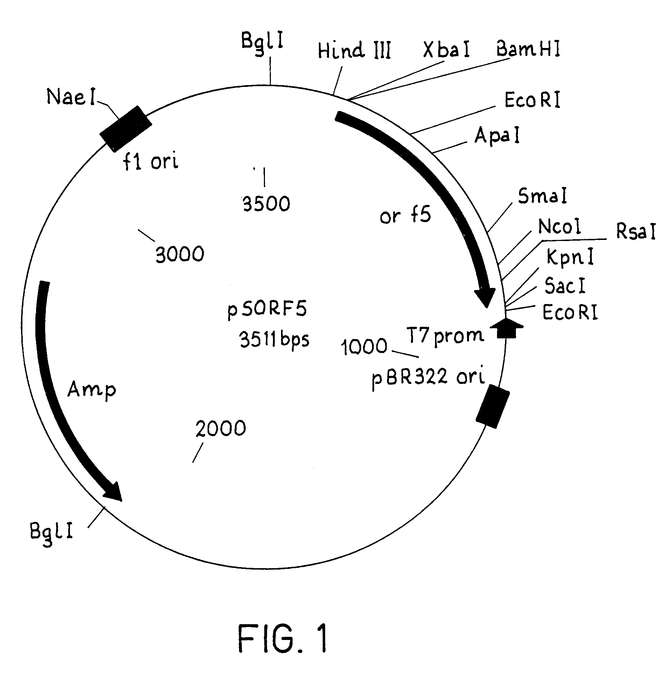

The 640 bp band (200 ng) was cloned into SmaI-cut pTZ18R (50 ng) (Pharmacia, Sweden) by using an overnight ligation at 15C with 1 Weiss unit of T4 ligase (Promega, WI) in 20 ul 1xT4 ligase buffer (Promega, WI). The ligation mixture (10 ul) was used to transform E.coli strain JM109 cells; competent JM109 cells were prepared as described by Sambrook et al., supra. The recombinant plasmid was denominated pSORF5 (Figure 1). The 640 bp insert was sequenced with a Taq polymerase sequencing (Promega, WI).

EXAMPLE 2 Construction of Expression Plasmid pSORINδ

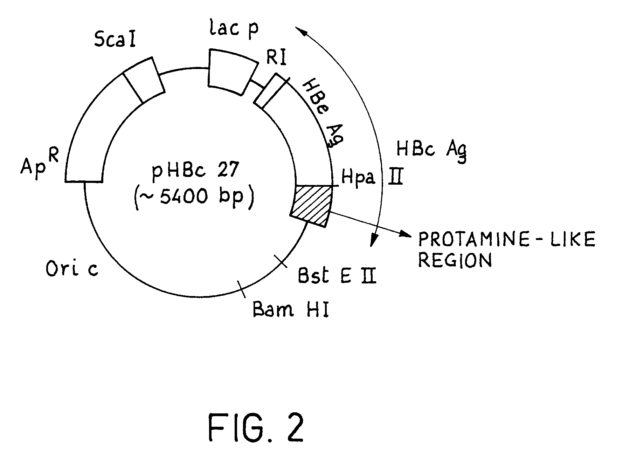

The expression plasmid pHBc27 (Figure 2) was used to provide appropriate transcription and other control elements for expression of HDAg. Any other equivalent expression vector is suitable. pHBc27, a derivative of pBR322, contains sequence encoding the hepatitis B virus (HBV) core antigen (HBcAg), and is described in Stahl et al., Proc. Natl. Acad. Sci. USA 79:1601 (1982). A lac promoter carrying the UV5 mutation (lac UV5 promoter), the ribosomal binding site (RBS) of beta-galactosidase, and sequence encoding the first eight amino acids of beta-galactosidase are located immediately upstream of the EcoR1 site in pHBc27. The BamH1 site in pHBc27 is located within the gene for tetracycline resistance (tet), approximately 350 bases pairs (bp) from the 3' end of the tet gene.

The plasmid HBc27 was cut with EcoR1 and BamH1 and the large fragment (pLac, approximately 4,500 bp) containing transcription and other control elements was purified from the short fragment (1,011 bp) containing the HBcAg sequence. The EcoR1 and BamH1 ends of pLac were filled in (blunt ended) with the Klenow fragment of E.coli DNA polymerase according to standard methods (Sambrook et al., v. supra). pSORF5 provided HDAg sequence for expression of HDAg-like polypeptide. A BamH1/Rsal fragment of 627 bp encoding all but the carboxyl-terminal six amino acids of ORF5 was obtained from pSORF5, and blunt ended as described above.

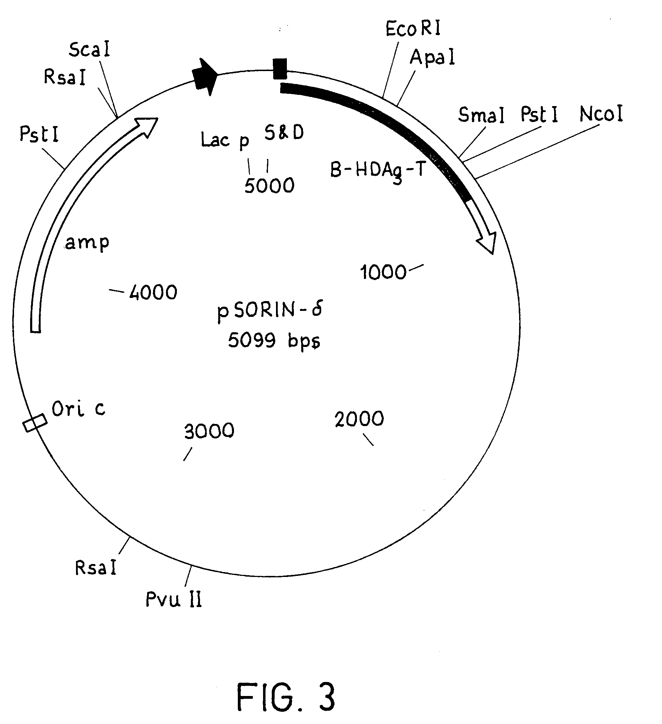

The 627 bp fragment from pSORF5 was cloned into pLac by blunt end ligation (see Sambrook et al., supra) and the correct orientation of the ORF5 insert with respect to transcriptional control elements was confirmed by restriction mapping. The resulting plasmid, pSORIN (Fig. 3, Table 2, deposited at DSM No .6774) was used to transform E.coli cells of strain W3 110, and 1.0 ml aliquots of cultures were frozen in 15% glycerol at -70C.E.coli strain W3 110 is deposited with the American Type Culture Collection (ATCC), No. 27325.

Table 2 <u>Nucleic acid sequence of pSORIN</u> .

```
************************************************************
* REPRESENTATION OF CLEAVAGE SITES ON THE SEQUENCE *
************************************************************

(+ LacUV5 promoter)
(* Bgalactosidase S&D)
(- orf5 gene + trailer sequence)



GCCGATTCATTAATGCTGGCACGACAGGTTTCCCGACTGGAAAGCGGGCA
+++++++++++++++++++++++++++++++++++++++++++++++++++


GTGAGCGCAACGCAATTAATGTGAGTTAGCTCACTCATTAGGCACCCCAG
+++++++++++++++++++++++++++++++++++++++++++++++++++
                   LacUV5 promoter

GCTTTACACATTTATGCTTCCATCATCGGCTCGTATAATGTGTGGAATTG
+++++++++++++++++++++++++++++++++++++++++++++++++++


TGAGCGGATAACAATTTCACACAGGAAACAGCTATGACCATGATTACGGA
++++++++++++++++++++++++*******
                        S&D       M  T  M  I  T  D

TTCACTGGAATTGATCCCCATGAGCCGGTCCGAGTCGAGGAAGAACCGCG
    S  L-----------------------------------------


GAGGGAGAGAAGAGATCCTCGAGCAGTGGGTGGCCGGAAGAAAGAAGTTA
------------------------------------------------
```

EP 0 485 347 B1

```
GAGGAACTCGAGAGAGACCTCCGGAAGACAAAGAAGAAACTCAAAAAGAT
--------------------------------------------------
                              E
                              c
                              o
                              R
                              I
TGAGGACGAAAATCCCTGGCTGGGGAACATCAAAGGAATTCTCGGAAAGA
--------------------------------------------------
                                           A
                                           p
                                           a
                                           I
AGGATAAGGATGGAGAGGGGGCTCCCCCGGCGAAGAGGGCCCGAACGGAC
--------------------------------------------------

CAGATGGAGGTAGACTCCGGACCTAGGAAGAGGCCTCTCAGGGGAGGATT  500
--------------------------------------------------
CACCGACAAGGAGAGGCAGGATCACCGACGAAGGAAGGCCCTCGAGAACA
--------------------------------------------------

AGAAGCAGCTATCGGCGGGAGGCAAAGAACCTCAGCAAGGAGGAAGAAGA
--------------------------------------------------

GGAACTCAGGAGGTTGACCGAGGAAGACGAGAGAAGGGAAAGAAGAGTAG
--------------------------------------------------

CCGGCCCGCCGGTTGGGGGTGTGATCCCCCTCGAAGGTGGATCGAGGGGA
--------------------------------------------------
            S                         P
            m                         s
            a                         t
            I                         I
GCGCCCGGGGGCGGCTTCGTCCCCAGTCTGCAGGGAGTCCCGGAGTCCCC
--------------------------------------------------
                                           N
                                           c
                                           o
                                           I
CTTCTCTCGGACCGGGGAGGGGCTGGACATCAGGGGAAACCGGGGATTTC
--------------------------------------------------

CATGGGATATACTCTTCCCAGCCGATCCGCCCTTTTCTCCCCAGAGGGGT
--------------------------------------------------

CCTCTACGCCGGAGCCATCGTGGCCGGCATCACCGGCGCCACAGGTGCGG
--------------------------------------------------

TTGCTGGCGCCTATATCGCCGACATCACCGATGCGGAAGATCGGGCTCGC
--------------------------------------------------

CACTTCGGGCTCATGAGCGCTTGTTTCGGCGTGGGTATGGTGGCAGGCCC  1000
--------------------------------------------------
```

```
CGTGGCCGGGGGACTGTTGGGCGCCATCTCCTTGCATGCACCATTCCTTG
--------------------------------------------------

CGGCGGCGGTGCTCAACGGCCTCAACCTACTACTGGGCTGCTTCTTAATG
--------------------------------------------------
CAGGAGTCGCATAAGATCCTCTACGCCGGACGCATCGTGGCCGGCATCAC
--------------

CGGCGCCACAGGTGCGGTTGCTGGCGCCTATATCGCCGACATCACCGATG

GGGAAGATCGGGCTCGCCACTTCGGGCTCATGAGCGCTTGTTTCGGCGTG

GGTATGGTGGCAGGCCCCGTGGCCGGGGCAGTGTTGGGCGCCATCTCCTT

GCATGCACCATTCCTTGCGGCGGCGGTGCTCAACGGCCTCAACCTACTAC

TGGGCTGCTTCCTAATGCAGGAGTCGCATAAGGGAGAGCGTCGACCGATG

CCCTTGAGAGCCTTCAACCCAGTCAGCTCCTTCCGGTGGCGCGGGCAT

GACTATCGTCGCCGCACTTATGACTGTCTTCTTTATCATGCAACTCGTAG 1500

GACAGGTGCCGGCAGCGCTCTGGGTCATTTTCGGCGAGGACCGCTTTCGC

TGGAGCGCGACGATGATCGGCCTGTCGCTTGCGGTATTCGGAATCTTGCA

CGCCCTCGCTCAAGCCTTCGTCACTGGTCCCGCCACCAAACGTTTCGGCG

AGAAGCAGGCCATTATCGCCGGCATGGCGGCCGACGCGCTGGGCTACGTC

TTGCTGGCGTTCGCGACGCGAGGCTGGATGGCCTTCCCCATTATGATTCT

TCTCGCTTCCGGCGGCATCGGGATGCCCGCGTTGCAGGCCATGCTGTCCA
```

11

GGCAGGTAGATGACGACCATCAGGGACAGCTTCAAGGATCGCTCGCGGCT

CTTACCAGCCTAACTTCGATCACTGGACCGCTGATCGTCACGGCGATTTA

TGCCGCCTCGGCGAGCACATGGAACGGGTTGGCATGGATTGTAGGCGCCG

CCCTATACCTTGTCTGCCTCCCCGCGTTGCGTCGCGGTGCATGGAGCCGG 2000

GCCACCTCGACCTGAATGGAAGCCGGCGGCACCTCGCTAACGGATTCACC

ACTCCAAGAATTGGAGCCAATCAATTCTTGCGGAGAACTGTGAATGCGCA

AACCAACCCTTGGCAGAACATATCCATCGCGTCCGCCATCTCCAGCAGCC

GCACGCGGCGCATCTCGGGCAGCGTTGGGTCCTGGCCACGGGTGCGCATG

ATCGTGCTCCTGTCGTTGAGGACCCGGCTAGGCTGGCGGGGTTGCCTTAC

TGGTTAGCAGAATGAATCACCGATACGCGAGCGAACGTGAAGCGACTGCT

GCTGCAAAACGTGTGCGACCTGAGCAACAACATGAATGGTCTTCGGTTTC

CGTGTTTCGTAAAGTCTGGAAACGCGGAAGTCAGCGCCCTGCACCATTAT

GTTCCGGATCTGCATCGCAGGATGCTGCTGGCTACCCTGTGGAACACCTA

CATCTGTATTAACGAAGCGCTGGCATTGACCCTGAGTGATTTTTCTCTGG 2500

TCCCGCCGCATCCATACCGCCAGTTGTTTACCCTCACAACGTTCCAGTAA

CCGGGCATGTTCATCATCAGTAACCCGTATCGTGAGCATCCTCTCTCGTT

TCATCGGTATCATTACCCCCATGAACAGAAATTCCCCCTTACACGGAGGC

ATCAAGTGACCAAACAGGAAAAAACCGCCCTTAACATGGCCCGCTTTATC

AGAAGCCAGACATTAACGCTTCTGGAGAAACTCAACGAGCTGGACGCGGA

TGAACAGGCAGACATCTGTGAATCGCTTCACGACCACGCTGATGAGCTTT
```
            P
            v
            u
            2
```
ACCGCAGCTGCCTCGCGCGTTTCGGTGATGACGGTGAAAACCTCTGACAC

ATGCAGCTCCCGGAGACGGTCACAGCTTGTCTGTAAGCGGATGCCGGGAG

CAGACAAGCCCGTCAGGGCGCGTCAGCGGGTGTTGGCGGGTGTCGGGGCG

CAGCCATGACCCAGTCACGTAGCGATAGCGGAGTGTATACTGGCTTAACT 3000
```
              R
              s
              a
              I
```
ATGCGGCATCAGAGCAGATTGTACTGAGAGTGCACCATATGCGGTGTGAA

ATACCGCACAGATGCGTAAGGAGAAAATACCGCATCAGGCGCTCTTCCGC

TTCCTCGCTCACTGACTCGCTGCGCTCGGTCGTTCGGCTCCGGCGAGCGG

TATCAGCTCACTCAAAGGCGGTAATACGGTTATCCACAGAATCAGGGGAT

AACGCAGGAAAGAACATGTGAGCAAAAGGCCAGCAAAAGGCCAGGAACCG

TAAAAAGGCCGCGTTGCTGGCGTTTTTCCATAGGCTCCGCCCCCCTGACG

AGCATCACAAAAATCGACGCTCAAGTCAGAGGTGGCGAAACCCGACAGGA

CTATAAAGATACCAGGCGTTTCCCCCTGGAAGCTCCCTCGTGCGCTCTCC

TGTTCCGACCCTGCCGCTTACCGGATACCTGTCCGCCTTTCTCCCTTCGG

GAAGCGTGGCGCTTTCTCAATGCTCACGCTGTAGGTATCTCAGTTCGGTG 3500

TAGGTCGTTCGCTCCAAGCTGGGCTGTGTGCACGAACCCCCCGTTCAGCC

CGACCGCTGCGCCTTATCCGGTAACTATCGTCTTGAGTCCAACCCGGTAA

GACACGACTTATCGCCACTGGCAGCAGCCACTGGTAACAGGATTAGCAGA

GCGAGGTATGTAGGCGGTGCTACAGAGTTCTTGAAGTGGTGGCCTAACTA

CGGCTACACTAGAAGGACAGTATTTGGTATCTGCGCTCTGCTGAAGCCAG

TTACCTTCGGAAAAAGAGTTGGTAGCTCTTGATCCGGCAAACAAACCACC

GCTGGTAGCGGTGGTTTTTTTGTTTGCAAGCAGCAGATTACGCGCAGAAA

AAAAGGATCTCAAGAAGATCCTTTGATCTTTTCTACGGGGTCTGACGCTC

AGTGGAACGAAAACTCACGTTAAGGGATTTTGGTCATGAGATTATCAAAA

AGGATCTTCACCTAGATCCTTTTAAATTAAAAATGAAGTTTTAAATCAAT 4000

CTAAAGTATATATGAGTAAACTTGGTCTGACAGTTACCAATGCTTAATCA

GTGAGGCACCTATCTCAGCGATCTGTCTATTTCGTTCATCCATAGTTGCC

TGACTCCCCGTCGTGTAGATAACTACGATACGGGAGGGCTTACCATCTGG

CCCCAGTGCTGCAATGATACCGCGAGACCCACGCTCACCGGCTCCAGATT

TATCAGCAATAAACCAGCCAGCCGGAAGGGCCGAGCGCAGAAGTGGTCCT

GCAACTTTATCCGCCTCCATCCAGTCTATTAATTGTTGCCGGGAAGCTAG

AGTAAGTAGTTCGCCAGTTAATAGTTTGCGCAACGTTGTTGCCATTGCTG

P
s
t
I
CAGGCATCGTGGTGTCACGCTCGTCGTTTGGTATGGCTTCATTCAGCTCC

GGTTCCCAACGATCAAGGCGAGTTACATGATCCCCCATGTTGTGCAAAAA

AGCGGTTAGCTCCTTCGGTCCTCCGATCGTTGTCAGAAGTAAGTTGGCCG 4500

CAGTGTTATCACTCATGGTTATGGCAGCACTGCATAATTCTCTTACTGTC

ATGCCATCCGTAAGATGCTTTTCTGTGACTGGTGAGTACTCAACCAAGTC

ATTCTGAGAATAGTGTATGCGGCGACCGAGTTGCTCTTGCCCGGCGTCAA

CACGGGATAATACCGCGCCACATAGCAGAACTTTAAAAGTGCTCATCATT

GGAAAACGTTCTTCGGGGCGAAAACTCTCAAGGATCTTACCGCTGTTGAG

ATCCAGTTCGATGTAACCCACTCGTGCACCCAACTGATCTTCAGCATCTT

TTACTTTCACCAGCGTTTCTGGGTGAGCAAAAACAGGAAGGCAAAATGCC

GCAAAAAAGGGAATAAGGCCGACACGGAAATGTTGAATACTCATACTCTT

CCTTTTTCAATATTATTGAAGCATTTATCAGGGTTATTGTCTCATGAGCG

GATACATATTTGAATGTATTTAGAAAAATAAACAAATAGGGGTTCCGCGC 5000

ACATTTCCCCGAAAAGTGCCACCTGACGTCTAAGAAACCATTATTATCAT

GACATTAACCTATAAAAATAGGCGTATCACGAGGCCCTTTCGTCTTCAA

Given the structure of pSORINδ, the polypeptide expected to be expressed from pSORINδ consists of the amino acid sequence shown in Table 1, beginning with the amino terminal portion and progressing to the carboxyl terminal portion of the polypeptide: a) eight amino acids derived from beta galactosidase, b) four amino acids derived from linker sequence used in construction of the respective plasmids, c) 209 amino acids derived from ORF5 (HDAg) and d) 89 amino acids derived from the tet gene of pBR322. The eight amino acids derived from beta-galactosidase are hereinafter termed the "B" region of the expressed polypeptide. The 89 amino acids derived from the tet gene, expected to display a secondary structure including "turn" and "extended" regions, is hereinafter termed the "T" or "trailer" region. The full-length expressed polypeptide therefore comprises B, HDAg and T amino acids, and is hereinafter termed the "B-HDAg-T" polypeptide and is the preferred form of HDAg-like polypeptide.

EXAMPLE 3 Growth of Cells Transformed with pSORINδ

A 1 ml frozen aliquot of stock E.coli strain W3 110 transformed with pSORIN was thawed and grown at 37C for 16 hours in Luria-Bertani broth with 50 ug/ml ampicillin. The overnight culture was inoculated into a 14-liter working volume fermenter (Chemag, F23000) and grown under the following constant conditions:
 a) Stirring rate: 400 rpm.
 b) Air flow rate: 2 liters/min.
 c) Temperature: 37C.
 d) pO2: 60%.
 e) pH: 7.4.
The bacterial growth was monitored at 590 nm. When the culture had attained an optical density (OD) of 0.5, isopropyl-B-D-galactoside (IPTG) was added to the culture at a final concentration of 1.0 mM in order to induce expression of B-HDAg-T under control of the lac UV5 promoter. Thereafter, the temperature was reduced to 30C and the stirring rate was increased to 1,300 rpm. The cells were harvested three hours post-induction with IPTG by pelleting at 3,000xg for 10 min. at 4C.

EXAMPLE 4 Purification of B-HDAg-T Polypeptide by Sucrose Density Gradient Centrifugation

Pelleted E.coli cells as described above were resuspended in 50 mM Tris/HCl pH 8.0, 1.0 mM EDTA, 1.0 mM FMSF and 100 mM NaCl, then disrupted by sonication (Braun Labsonic Model 1510 sonicator; three bursts of 60 sec. each at 100W, on ice). Following sonication, 8.0 mg/ml of sodium deoxycholate were then added at 4C under mixing. The cellular membranes, inclusion bodies and other cellular debris were removed by pelleting at 10,000 rpm in a JA17 Beckman rotor for 30 min. at 4C. The supernatant was loaded on a 10-50% sucrose gradient made with 50 mM Tris/HCl pH 7.5 and the gradient centrifuged at 35,000 rpm in a SW40 TI Beckman rotor, for 16 hours at 4C. Contents of the gradient were recovered at a flow rate of 0.8 ml/min. and collected as 0.4 ml fractions. The B-HDAg-T polypeptide was found at the bottom of the gradient in the form of multimeric aggregates. The purity of the B-HDAg-T material from the gradient is estimated at 85-90% based on sodium dodecyl sulfate polyacrylamide gel electrophoresis (SDS PAGE) and staining of polypeptide bands with Coomassie brilliant blue dye. In other words, no more than 10-15% non-B-HDAg-T polypeptide is present in the isolated fractions taken from the bottom of the gradient.
A sample of the B-HDAg-T polypeptide material obtained from sucrose gradients as described above was stained with uranyl acetate and examined with electron microscopy. The multimeric forms of HDAg obtained from the sucrose gradients are generally larger than 10. daltons The multimeric aggregates consist of nucleoprotein complexes of B-HDAg-T and RNA. It is known that HDAg is capable of binding to RNA. Chang et al., J. Virol. 62:2403 (1988). Further, the applicants have observed that sedimentation of HDAg particles (aggregates) is not observed if the E.coli lysate is treated with RNAase prior to loading on the sucrose gradient (data not shown).

EXAMPLE 5 Purification of B-HDAg-T Polypeptide by immunoaffinity Chromatography

As an alternative to purification of B-HDAg-T by sucrose gradient centrifugation, a double immunoaffinity column procedure was developed allowing selection specifically of full-length B-HDAg-T polypeptide. The first immunoaffinity column comprised Sepharose-CNBr (Pharnmacia, Sweden) coupled with a monoclonal antibody raised against the...-galactosidase (B) region of B-HDAg-T according to standard techniques (MAB-B). Coupling of antibody to the Sepharose-CNBr was performed according to the procedures recommended by Pharmacia, with a coupling yield in excess of 95%. The second immunoaffinity column comprised Sepharose-CNBr coupled with a monoclonal antibody raised against the T region of the B-HDAg-T polypeptide, according to standard techniques (MAB-T). Specifically, MAB-T is specific for the sequence WRLYRRH present in the T region of B-HDAg-T.
The same material as was applied to the top of the sucrose gradients, i.e., the crude sonicated lysate following centrifugation at 10,000 rpm for 10 min. at 4C ad described above, was applied to the

EP 0 485 347 B1

immunoaffinity column containing MAB-B coupled with Sepharose-CNBr. MAB-B-bound material was eluted with high salt buffer comprising 3.5M MgCl2, 10 mM phosphate pH 7.2, then dialysed twice against 1 liter of phosphate buffered saline (PBS). The eluted material was then applied to the second immunoaffinity column containing MAB-T and eluted again with high salt buffer. The material eluted following the second immunoaffinity purification was determined to be full-length B-HDAg-T of 98% purity as determined by SDS PAGE and Coomassie Blue staining.

EXAMPLE 6 Competitive Immunoassay for Total Immunoglobulin (Ig) Anti-HDAg

Microtiter plates were coated with MAB-T (anti-WRLYRRH) using standard procedures. Then HDAg in the form of the expressed and sucrose gradient-purified B-HDAg-T polypeptide as described above were added to the plates at a concentration of 0.2 ug/ml. The plates that had been coated with B-HDAg-T were incubated at room temperature for 18 hours, then washed with BPS. Following washing, the plates were suitable for use in determination of total Ig anti-HDV in blood sera.

Each well of the microtiter plates treated as above received 10 ul of serum to be analyzed. Immediately following addition of test serum, an anti-HDAg polyclonal antibody preparation labelled with horseradish peroxidase (HRP) was added to each well. Following three hours of incubation at 37C, the reagents were washed out and the quantity of anti-HDV polyclonal antibody bound to the plate was observed by addition of H2O2 and tetramethylbenzidine. The colorimetric reaction was stopped with 1.0 M sulfuric acid and the absorbance at 450 nm and 630 nm was measured. In this competitive assay format, the amount of anti-HDAg polyclonal antibody bound to the microtiter plate, as measured by the colorimetric reaction with HRP, is inversely proportional to the amount of anti-HDAg antibody in the serum sample.

EXAMPLE 7 Immunoassay for Determination of IgM ant-HDV

Microtiter plates were coated with anti-human IgM monoclonal antibody according to standard methods. Sera to be tested (10 ul) were added to the plate wells and incubated for one hour at 37C. Following this, anti-HDAg polyclonal antibody labelled with HRP and the expressed, sucrose gradient-purified B-HDAg-T polypeptide as described above were added to the plates and incubated for two hours at 37C. Following this incubation, the unbound reagents were removed by washing and the quantity of HRP-labelled antibody bound to the plates was observed by performing the colorimetric reaction as described above.

**Claims**

1. A composition comprising a multimeric aggregate able to raise antibody against hepatitis D antigen (HDAg), said multimeric aggregate comprising recombinant polypeptides, wherein said polypeptides comprise:
   a first aminoacid sequence having an antigenicity of HDAg;
   a second aminoacid sequence at the amino-terminal of said first aminoacid sequence and a third aminoacid sequence at the carboxy-terminal of said first aminoacid sequence, said second and said third amino acid sequences being heterologous to the sequence of HDAg;
   said second aminoacid sequence having the following sequence:
   M T M I T D S L
   said third aminocid sequence having the following sequence:

   R G P L R R S H R G R H H R R H R C G

   C W R L Y R R H H R C G R S G S P L R

   A H E R L F R R G Y G G R P R G R G T

   V G R H L L A C T I P C G G G A Q R P

   Q P T T G L L L N A G V A

2. The composition according to claim 1 wherein said polypeptides comprise the following aminoacid sequence:

18

```
M  T  M  I  T  D  S  L  E  L  I  P  M  S  R  S  E  S  R  K

N  R  G  G  R  E  E  I  L  E  Q  W  V  A  G  R  K  K  L  E

E  L  E  R  D  L  R  K  T  K  K  K  L  K  K  I  E  D  E  N

P  W  L  G  N  I  K  G  I  L  G  K  K  D  K  D  G  E  G  A

P  P  A  K  R  A  R  T  D  Q  M  E  V  D  S  G  P  R  K  R

P  L  R  G  G  F  T  D  K  E  R  Q  D  H  R  R  R  K  A  L

E  N  K  K  K  Q  L  S  A  G  G  K  N  L  S  K  E  E  E  E

E  L  R  R  L  T  E  E  D  E  R  R  E  R  R  V  A  G  P  P


V  G  G  V  I  P  L  E  G  G  S  R  G  A  P  G  G  G  F  V

P  S  L  Q  G  V  P  E  S  P  F  S  R  T  G  E  G  L  D  I

R  G  N  R  G  F  P  W  D  I  L  F  P  A  D  P  P  F  S  P

Q  R  G  P  L  R  R  S  H  R  G  R  H  H  R  R  H  R  C  G

C  W  R  L  Y  R  R  H  H  R  C  G  R  S  G  S  P  L  R  A

H  E  R  L  F  R  R  G  Y  G  G  R  P  R  G  R  G  T  V  G

R  H  L  L  A  C  T  I  P  C  G  G  A  Q  R  P  Q  P  T

T  G  L  L  L  N  A  G  V  A  -
```

3. The composition according to any of previous claims wherein said multimeric aggregate comprises RNA.

4. An immunoassay kit comprising as specific reagent the composition according to any of previous claims.

5. A method of immunoassay of an analyte antibody specific to HDAg in a sample comprising the steps of:
    reacting said sample with the composition of any of claims from 1 to 3 to get the binding thereof with said analyte antibody;
    detecting said binding.

6. A method of immunoassay of an analyte HDAg in a sample comprising the steps of:
    competitive binding to an antibody specific to HDAg of said analyte antigen and of the composition of any of claims from 1 to 3;
    detecting said competitive binding.

7. A process of recovering multimeric aggregate according to claim from 1 to 3 from host cells comprising the steps of:

disrupting said host cells by sonication;

separating said aggregates from cell membrane material and cell inclusion bodies by centrifugation.

8. A process of recovering multimeric aggregate according to claim from 1 to 3 from host cells comprising the steps of:

disrupting said host cells by sonication;

separating said aggregates from cell membrane material and cell inclusion bodies by centrifugation;

further separating said aggregates from cytosolic material by sequential immunoaffinity chromatography specific to said second aminoacid sequence and to said third aminoacid sequence.

9. A nucleic acid coding for the polypetide of claim 3 having the following sequence:

```
ATGACCATGATTACGGATTCACTGGAATTGATCCCCATGAGCCGGTCCGAGTCGAGGAAG

AACCGCGGAGGGAGAGAAGAGATCCTCGAGCAGTGGGTGGCCGGAAGAAAGAAGTTAGAG

GAACTCGAGAGAGACCTCCGGAAGACAAAGAAGAAACTCAAAAAGATTGAGGACGAAAT

CCCTGGCTGGGGAACATCAAAGGAATTCTCGGAAAGAAGGATAAGGATGGAGAGGGGGCT

CCCCCGGCGAAGAGGGCCCGAACGGACCAGATGGAGGTAGACTCCGGACCTAGGAAGAGG

CCTCTCAGGGGAGGATTCACCGACAAGGAGAGGCAGGATCACCGACAAGGAAGGCCCTC

GAGAACAAGAAGAAGCAGCTATCGGCGGGAGGCAAGAACCTCAGCAAGGAGGAAGAAGAG

GAACTCAGGAGGTTGACCGAGGAAGACGAGAGAAGGGAAAGAAGAGTAGCCGGCCCGCCG

GTTGGGGGTGTGATCCCCCTCGAAGGTGGATCGAGGGGAGCGCCCGGGGGCGGCTTCGTC

CCCAGTCTGCAGGGAGTCCCGGAGTCCCCCTTCTCTCGGACCGGGGAGGGGCTGGACATC

AGGGGAAACCGGGGATTTCCATGGATATACTCTTCCCAGCCGATCCGCCCTTTTCTCCC

CAGAGGGGTCCTCTACGCCGGAGCCATCGTGGCCGGCATCACCGGCGCCACAGGTGCGGT

TGCTGGCGCCTATATCGCCGACATCACCGATGCGGAAGATCGGGCTCGCCACTTCGGGCT

CATGAGCGCTTGTTTCGGCGTGGGTATGGTGGCAGGCCCCGTGGCCGGGGGACTGTTGGG

CGCCATCTCCTTGCATGCACCATTCCTTGCGGCGGCGGTGCTCAACGGCCTCAACCTACT

ACTGGGCTGCTTCTTAATGCAGGAGTCGCATAA.
```

**Patentansprüche**

1. Multimerisches Aggregat enthaltende Verbindung, im Grade Antikoerper gegen Hepatitis D (HDAg) zu entlassen, wobei das multimerische Aggregat rekombinierbare Polypeptide enthaelt und wobei diese Polypeptide folgende Sequenzen aufweisen:

eine erste Aminosaeurensequenz mit einer Antigenitaet von HDAg;

eine zweite Aminosaeurensequenz am Aminoterminal der ersten Aminosaeurensequenz und eine

20

dritte Aminosaeurensequenz am Carboxyterminal der ersten Aminosaeurensequenz, wobei die zweite und dritte Aminosaeurensequenzen zur HDAg-Sequenz heterologisch sind und wobei

die zweite Aminosaeurensequenz die folgende Sequenz:

M T M I T D S L

und die dritte Aminosaeurensequenz die folgende Sequenz:

R G P L R R S H R G R H H R R H R C G

C W R L Y R R H H R C G R S G S P L R

A H E R L F R R G Y G G R P R G R G T

V G R H L L A C T I P C G G G A Q R P

Q P T T G L L L N A G V A

aufweisen.

2. Verbindung nach Anspruch 1, worin die Polypeptide die folgende Aminosaeurensequenz aufweisen

M T M I T D S L E L I P M S R S E S R K

N R G G R E E I L E Q V V A G R K K L E

E L E R D L R K T K K K L K K I E D E N

P W L G N I K G I L G K K D K D G E G A

P P A K R A R T D Q M E V D S G P R K R

P L R G G F T D K E R Q D H R R R K A L

E N K K K Q L S A G G K N L S K E E E E

E L R R L T E E D E R R E R R V A G P P

```
V   G   G   V   I   P   L   E   G   G   S   R   G   A   P   G   G   G   F   V

P   S   L   Q   G   V   P   E   S   P   F   S   R   T   G   E   G   L   D   I

R   G   N   R   G   F   P   W   D   I   L   F   P   A   D   P   P   F   S   P

Q   R   G   P   L   R   R   S   H   R   G   R   H   H   R   R   H   R   C   G

C   W   R   L   Y   R   R   H   H   R   C   G   R   S   G   S   P   L   R   A

H   E   R   L   F   R   R   G   Y   G   G   R   P   R   G   R   G   T   V   G

R   H   L   L   A   C   T   I   P   C   G   G   G   A   Q   R   P   Q   P   T

T   G   L   L   L   N   A   G   V   A   -
```

3. Verbindung nach je einem der vorangehenden Ansprueche, worin das multimerische Aggregat RNA enthaelt.

4. Immunoassay-Ausruestung, die als spezifisches Reagens die Verbindung nach je einem der vorangehenden Ansprueche enthaelt.

5. Immunoassay-Verfahren fuer einen fuer das HDAg spezifischen Analyte-Antikoerper in einem Muster, mit folgenden Verfahrensschritten:
   Reaktion des Musters mit der Verbindung nach je einem der vorangehenden Ansprueche 1 bis 3 zur Erhaltung der Bindung desselben mit dem Analyte-Antikoerper;
   Ermittlung dieser Bindung.

6. Immunoassay-Verfahren fuer ein Analyte-HDAg in einem Muster, mit folgenden Verfahrensschritten:
   Saettigung mit einem zum HDAg des Analyte-Antigens und zur Verbindung nach je einem der Ansprueche 1 bis 3 spezifischen Antikoerper,
   Ermittlung dieser Saettigung.

7. Verfahren zur Rueckgewinnung eines multimerischen Aggregats nach je einem der Ansprueche 1 bis 3 aus Gastzellen, mit folgenden Verfahrensschritten:
   Aufbrechen der Gastzellen durch Sonifikation;
   Abtrennen des Aggregats von Zellemembranestoff und Zelleneinschlusskoerper durch Zentrifugieren.

8. Verfahren zur Rueckgewinnung eines multimerischen Aggregats nach den Anspruechen 1 bis 3 aus Gastzellen, mit folgenden Verfahrensschritten:
   Aufbrechen der Gastzellen durch Sonifikation;
   Abtrennen des Aggregats von Zellemembranestoff und Zelleneinschlusskoerper durch Zentrifugation;
   weitere Abtrennung der Aggregate vom cytosolischen Stoff durch Immunoaffinitaet-Sequenz-Chromatographie, die fuer die zweite und dritte Aminosaeurensequenz spezifisch ist.

9. Codierung von Nucleinsaeuren fuer die Polypeptide nach Anspruch 3, die die folgende Sequenz haben:

```
ATGACCATGATTACGGATTCACTGGAATTGATCCCCATGAGCCGGTCCGAGTCGAGGAAG
AACCGCGGAGGGAGAGAAGAGATCCTCGAGCAGTGGGTGGCCGGAAGAAAGAAGTTAGAG
GAACTCGAGAGAGACCTCCGGAAGACAAAGAAGAAACTCAAAAAGATTGAGGACGAAAAT
CCCTGGCTGGGGAACATCAAAGGAATTCTCGGAAAGAAGGATAAGGATGGAGAGGGGGCT
CCCCCGGCGAAGAGGGCCCGAACGGACCAGATGGAGGTAGACTCCGGACCTAGGAAGAGG
CCTCTCAGGGGAGGATTCACCGACAAGGAGAGGCAGGATCACCGACCAAGGAAGGCCCTC
GAGAACAAGAAGAAGCAGCTATCGGCGGGAGGCAAGAACCTCAGCAAGGAGGAAGAAGAG
GAACTCAGGAGGTTGACCGAGGAAGACGAGAGAAGGGAAAGAAGAGTAGCCGGCCCGCCG
GTTGGGGGTGTGATCCCCCTCGAAGGTGGATCGAGGGGAGCGCCCGGGGGCGGCTTCGTC
CCCAGTCTGCAGGGAGTCCCGGAGTCCCCCTTCTCTCGGACCGGGGAGGGGCTGGACATC
AGGGGAAACCGGGGATTTCCATGGGATATACTCTTCCCAGCCGATCCGCCCTTTTCTCCC
CAGAGGGGTCCTCTACGCCGGAGCCATCGTGGCCGGCATCACCGGCGCCACAGGTGCGGT
TGCTGGCGCCTATATCGCCGACATCACCGATGCGGAAGATCGGGCTCGCCACTTCGGGCT
CATGAGCGCTTGTTTCGGCGTGGGTATGGTGGCAGGCCCCGTGGCCGGGGGACTGTTGGG
CGCCATCTCCTTGCATGCACCATTCCTTGCGGCGGCGGTGCTCAACGGCCTCAACCTACT
ACTGGGCTGCTTCTTAATGCAGGAGTCGCATAA
```

**Revendications**

1. Composition comprenant un agrégate multimèrique capable de relâcher anticorps contre hépatite D antigène (HDAg), ledit agrégate multimèrique comprenant polypéptides de recombination, dans laquelle lesdits polypèptides comprendent:

une premiere séquence des aminoacides ayant une antigènicité de HDAg;

une deuxiéme séquence des aminoacides au amino-terminal de ladite premiere séquence des aminoacides et une sèquence troisième des aminoacides au carboxy-terminal de ladite premiere séquence des aminoacides, ladite deuxiéme et ladite troisième sequences des aminoacides étant hétérologue à la séquence de HDAg;

ladite deuxième séquence des aminoacides ayant la séquence suivante:

M T M I T D S L

ladite troisième séquence des aminoacides ayant le séquence suivante:

R G P L R R S H R G R H H R R H R C S

C W R L Y R R H H R C G R S G S P L R

A H E R L F R R G Y G G R P R G R G T

V G R H L L A C T I P C G G G A Q R P

Q P T T G L L L N A G V A

**2.** Composition selon la revendication 1, dans laquelle lesdites péptides comprendent les suivantes séquences des aminoacides:

```
M  T  M  I  T  D  S  L  E  L  I  P  M  S  R  S  E  S  R  K

N  R  G  G  R  E  E  I  L  E  Q  W  V  A  G  R  K  K  L  E

E  L  E  R  D  L  R  K  T  K  K  K  L  K  K  I  E  D  E  N

P  W  L  G  N  I  K  G  I  L  G  K  K  D  K  D  G  E  G  A

P  P  A  K  R  A  R  T  D  Q  M  E  V  D  S  G  P  R  K  R

P  L  R  G  G  F  T  D  K  E  R  Q  D  H  R  R  R  K  A  L

E  N  K  K  K  Q  L  S  A  G  G  K  N  L  S  K  E  E  E  E

E  L  R  R  L  T  E  E  D  E  R  R  E  R  R  V  A  G  P  P


V  G  G  V  I  P  L  E  G  G  S  R  G  A  P  G  G  G  F  V

P  S  L  Q  G  V  P  E  S  P  F  S  R  T  G  E  G  L  D  I

R  G  N  R  G  F  P  W  D  I  L  F  P  A  D  P  P  F  S  P

Q  R  G  P  L  R  R  S  H  R  G  R  H  H  R  R  H  R  C  G

C  W  R  L  Y  R  R  H  H  R  C  G  R  S  G  S  P  L  R  A

H  E  R  L  F  R  R  G  Y  G  G  R  P  R  G  R  G  T  V  G

R  H  L  L  A  C  T  I  P  C  G  G  G  A  Q  R  P  Q  P  T

T  G  L  L  L  N  A  G  V  A  -
```

**3.** Composition selon quelconque une des revendications précédentes, dans laquelle ledit agrégate multimerique comprend RNA.

**4.** Equipment de immunoassay, comprenant comme réactif spécifique la composition selon une quelconque des revendications précédentes.

**5.** Méthode d'immunoassay d'un anticorps d'analyte spécifique à HDAg dans un champion, comprenant les phases de:
faire réagir ledit champion avec la composition selon un quelconque des revendications 1 à 3 pour obtenir son liaison avec ledit anticorps d'analyte;
détecter ladite liaison.

6. Méthode d'immunoassay d'un analyte HDAg dans un champion, comprenant les phases de:
liaison compétitive à un anticorps spécifique à HDAg dudit analyte antigène et de la composition selon une quelconque des revendications 1 à 3;
détection de ladite liaison competitive.

7. Procédé de récupération d'agrégate multimèrique selon les revendications 1 à 3 de cellules hôtes, comprenant les phases de:
casser par sonification lesdites cellules hôtes;
séparer par sonification lesdite agrégates de matériel de membrane des cellules et de corps d'inclusion des cellules.

8. Procéde de récupération d'agrégate multimèrique selon les revendications 1 à 3 de cellules hôtes, comprenant les phases de:
casser par sonification lesdites cellules hôtes;
séparer par centrifugation lesdits agrégates de matériel de membrane des cellules et de corps d'inclusion des cellules;
séparer ultériorement leadits agrégates de matériel cytosolique par chromatographie d'immunoaffinité séquentiale spécifique à ladite deuxième séquence des aminoacides et à la troisième séquence des aminoacides.

9. Codification d'acide nucleique pour le polypéptide de la revendication 3 ayant la séquence suivantes:

ATGACCATGATTACGGATTCACTGGAATTGATCCCCATGAGCCGGTCCGAGTCGAGGAAG

AACCGCGGAGGGAGAGAAGAGATCCTCGAGCAGTGGGTGGCCGGAAGAAAGAAGTTAGAG

GAACTCGAGAGAGACCTCCGGAAGACAAAGAAGAAACTCAAAAAGATTGAGGACGAAAAT

CCCTGGCTGGGGAACATCAAAGGAATTCTCGGAAAGAAGGATAAGGATGGAGAGGGGGCT

CCCCCGGCGAAGAGGGCCCGAACGGACCAGATGGAGGTAGACTCCGGACCTAGGAAGAGG

CCTCTCAGGGGAGGATTCACCGACAAGGAGAGGCAGGATCACCGACCAAGGAAGGCCCTC

GAGAACAAGAAGAAGCAGCTATCGGCGGGAGGCAAGAACCTCAGCAAGGAGGAAGAAGAG

GAACTCAGGAGGTTGACCGAGGAAGACGAGAGAAGGGAAAGAAGAGTAGCCGGCCCGCCG

GTTGGGGGTGTGATCCCCCTCGAAGGTGGATCGAGGGGAGCGCCCGGGGGCGGCTTCGTC

CCCAGTCTGCAGGGAGTCCCGGAGTCCCCCTTCTCTCGGACCGGGGAGGGGCTGGACATC

AGGGGAAACCGGGGATTTCCATGGGATATACTCTTCCCAGCCGATCCGCCCTTTTCTCCC

CAGAGGGGTCCTCTACGCCGGAGCCATCGTGGCCGGCATCACCGGCGCCACAGGTGCGGT

TGCTGGCGCCTATATCGCCGACATCACCGATGCGGAAGATCGGGCTCGCCACTTCGGGCT

CATGAGCGCTTGTTTCGGCGTGGGTATGGTGGCAGGCCCCGTGGCCGGGGGACTGTTGGG

CGCCATCTCCTTGCATGCACCATTCCTTGCGGCGGCGGTGCTCAACGGCCTCAACCTACT

ACTGGGCTGCTTCTTAATGCAGGAGTCGCATAA.

FIG. 1

FIG. 2

FIG. 3